# EUROPEAN PATENT SPECIFICATION

(11) **EP 4 486 434 B1**
(45) Date of publication and mention of the grant of the patent: **24.06.2026**
(21) Application number: 23717005.5
(22) Date of filing: 22.03.2023
(51) Int. Cl.: A61N 1/36, A61N 1/372, A61N 1/378, A61N 1/05

(54) **IMPLANTABLE PULSE GENERATOR CHARGING ALERTS**
LADEALARME FÜR IMPLANTIERBAREN IMPULSGENERATOR
GÉNÉRATEUR D'IMPULSIONS IMPLANTABLE CHARGEANT DES ALERTES

(30) Priority: 25.03.2022 US 202263269917 P
(43) Date of publication of application: 08.01.2025
(73) Proprietor: Boston Scientific Neuromodulation Corporation, Valencia, CA 91355 (US)
(72) Inventor: SRIVASTAVA, Kyle, Saint Paul, MN 55102 (US); HUYNH, Dat, Hollywood, CA 90046 (US)
(74) Representative: Vossius & Partner Patentanwälte Rechtsanwälte mbB
(86) International application number: PCT/US2023/064805
(87) International publication number: WO 2023/183838

(56) References cited:
- US-A1- 2010 222 847
- US-B2- 11 000 688
- US-B2- 9 929 584

## Description

### FIELD OF THE INVENTION

This application relates to Implantable Medical Devices (IMDs), generally, and more specifically to implantable neurostimulator devices having implantable pulse generators (IPGs).

### BACKGROUND

Implantable stimulation devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516,227. However, the present invention may find applicability in any implantable medical device system, including a Deep Brain Stimulation (DBS) system.

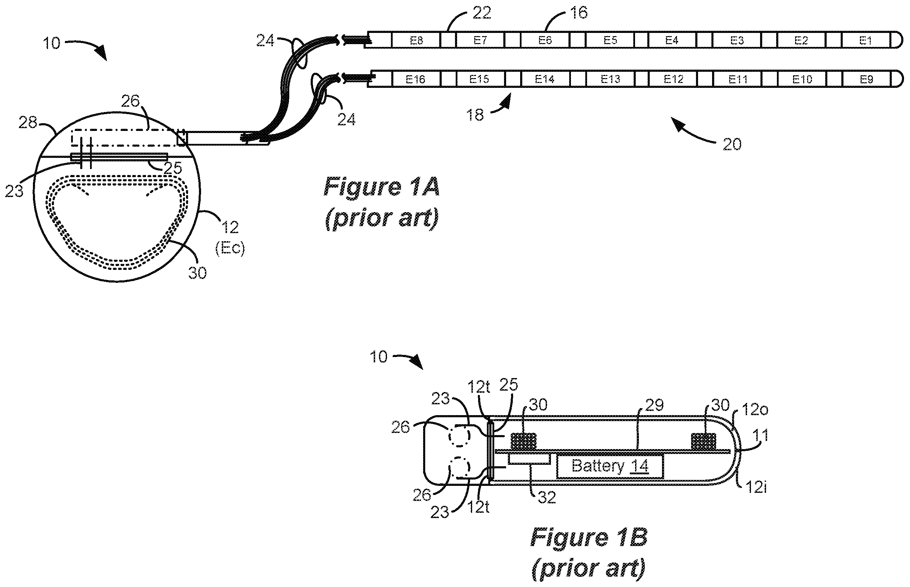

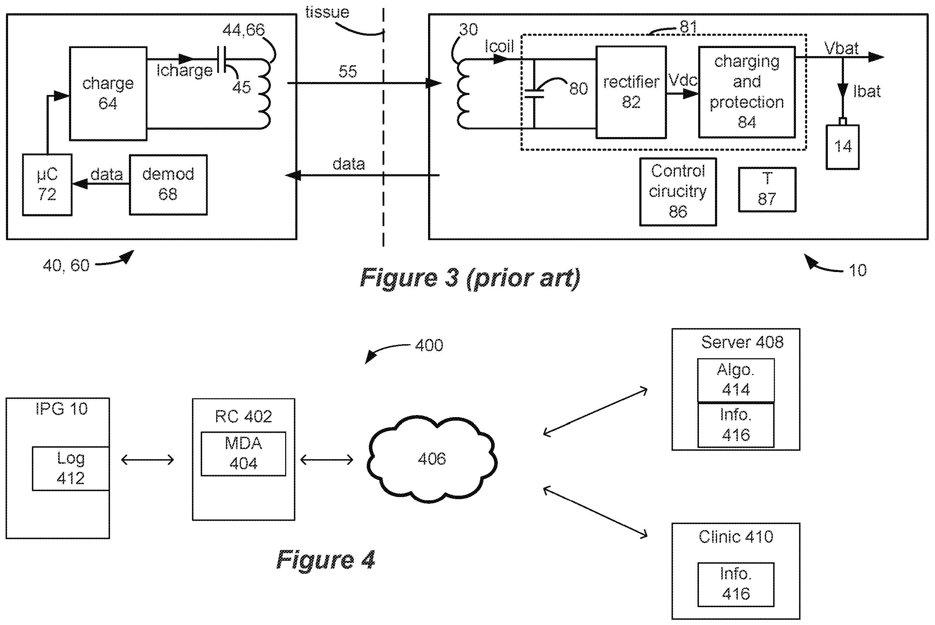

As shown in Figures 1A-1B, a SCS system typically includes an Implantable Pulse Generator (IPG) 10, which includes a biocompatible device case 12 formed of a conductive material such as titanium for example. The case 12 typically holds the control circuitry 86 (Fig. 3) and battery 14 (Fig. 1B) necessary for the TPG10 to function. The TPG10 is coupled to electrodes Ex 16 via one or more electrode leads 18, such that the electrodes 16 form an electrode array 20. The electrodes 16 are carried on a flexible body 22, which also houses the individual signal wires 24 coupled to each electrode. In the illustrated embodiment, there are eight electrodes (Ex) on two leads 18, although the number of leads and electrodes is application specific and therefore can vary. The leads 18 couple to lead connectors 26 in the IPG 10, which are fixed in a non-conductive header material 28 such as an epoxy. Feedthrough pins 23 connect to electrode contacts (not shown) in the lead connectors 26, which pins pass through a hermetic feedthrough 25 on the top of the case 12, where they are connected to stimulation circuitry inside of the IPG 10's case. Control circuitry 86 can include stimulation circuitry configured to provide stimulation current to selected ones of the electrodes, and can comprise circuitry disclosed for example in USPs 6,181,969, 8,606,362, 8,620,436, U.S. Patent Application Publications 2018/0071520 and 2019/0083796. The conductive case 12 material can also operate as a case electrode Ec to provide a return current path for currents provided at the lead based electrodes, Ex.

As shown in the cross-section of Figure 1B, the IPG 10 typically includes a printed circuit board (PCB) 29, along with various electronic components 32 mounted to the PCB 29, some of which are discussed subsequently. The IPG 10 traditionally includes a charging coil 30 for charging or recharging the IPG's battery 14 using an external charger. The case 12 is typically formed of two clam-shell-like portions 12i and 12o that that are designed when implanted to respectively face the inside and outside of the patient. These portions 12i and 12o are typically welded (11) together along the outer periphery of the case, and include flanges that are welded to the feedthrough 25. When so formed, the case 12 includes planar parallel major surfaces formed in the outside and inside case portions 12o and 12i, and a generally planar top surface 12t perpendicular to the major surfaces which includes the feedthrough 25.

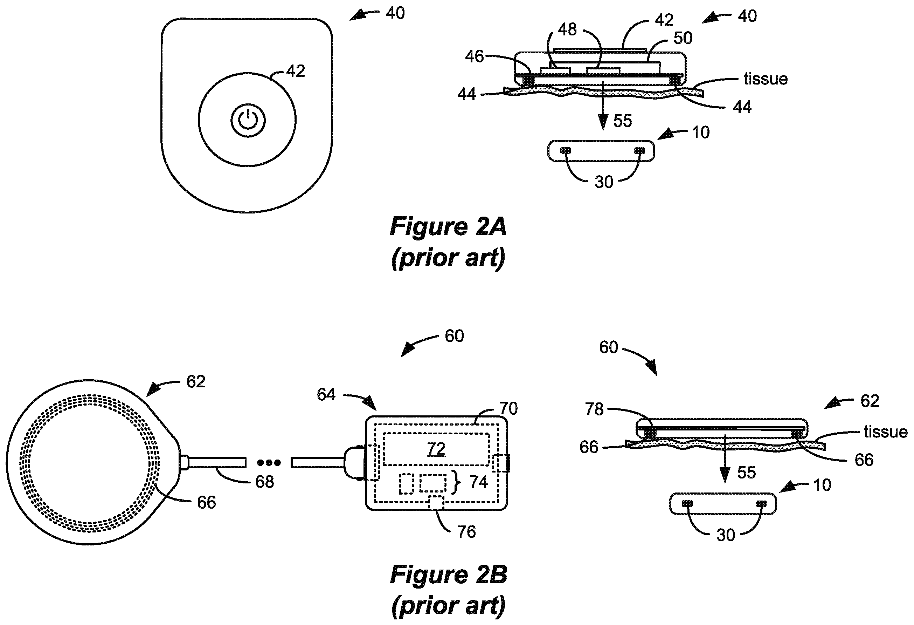

Figures 2A and 2B show the IPG 10 in communication with external chargers, and two different examples of chargers 40 and 60 are shown. Both types of chargers 40 and 60 are used to wirelessly convey power in the form of an electromagnetic field 55 (referred to as a "magnetic field" for short) to the IPG 10, which power can be used to recharge the IPG's battery 14. The transfer of power from external charger 40 is enabled by a primary charging coil 44 in Figure 2A, and by a primary charging coil 66 in Figure 2B. Figure 2A shows an example in which the charging coil 44 is integrated in the same housing as other charger electronics, while in Figure 2B the charging coil 66 and charger electronics are separated into different housings and connected by a cable 68.

In Figure 2A, the integrated charger 40 includes a PCB 46 on which electronic components 48 are placed, some of which are discussed subsequently. Charging coil 44 may be mounted to the PCB 46, and preferably on the side of the PCB that faces the IPG 10 as shown. A user interface, including touchable buttons, LEDs (not shown) and perhaps a display and a speaker (not shown), allows a patient or clinician to operate the external charger 40. In Figure 2A, the user interface is shown simply as including an on/off button 42 used to turn the magnetic field 55 on or off. A battery 50 provides power for the external charger 40, which battery 50 may itself be rechargeable. Charger 40 is typically configured to be hand-holdable and portable, and is described further in U.S. Patent Application Publication 2017/0361113.

In Figure 2B, the charger 60 comprises a charging coil assembly 62 and an electronics module 64 in separate housings which are connected by a cable 68. The charging coil assembly 62 includes the charging coil 66, while the electronics and user interface elements are provided by the electronics module 64. The electronics housing 64 may include a PCB 70, a battery 72, various control circuitry 74, and user interface elements 76 such as those mentioned above. Charger 60 despite generally being in two pieces 62 and 64 is also typically configured to be hand-holdable and portable, and is again described further in the above-referenced 2017/0361113 publication.

Transmission of the magnetic field 55 from either of chargers 40 or 60 to the IPG 10 occurs wirelessly and transcutaneously through a patient's tissue via inductive coupling. Figure 3 shows details of the circuitry used to implement such functionality. Primary charging coil 44 or 66 in the external charger is energized via charging circuit 64 with an AC current, Icharge, to create the AC magnetic charging field 55. A tuning capacitor 45 is provided to form a resonant LC tank with the charging coil 44 or 66, which generally sets the frequency of the AC magnetic field 55.

The magnetic portion of the electromagnetic field 55 induces a current Icoil in the secondary charging coil 30 within the IPG 10, which current is received at power reception circuitry 81. Power reception circuitry 81 can include a tuning capacitor 80, which is used to tune the resonance of the LC circuit in the IPG to the frequency of the magnetic field. One skilled will understand that the capacitors 45 or 80 may be placed in series or in parallel with their respective coils (inductances) 44/66 or 30, although it is preferred that the capacitor 45 be placed in series with the coil 44/66 in the charger 40/60, while the capacitor 80 is placed in parallel with the coil 30 in the IPG 10. The power reception circuitry 81 further includes a rectifier 82 used to convert AC voltage across the coil 30 to DC a DC voltage Vdc. Power reception circuitry 81 may further include other conditioning circuitry such as charging and protection circuitry 84 to generate a Voltage Vbat which can be used to provide regulated power to the IPG 10, and to generate a current Ibat which is used to charge the battery 14. The frequency of the magnetic field 55 can be perhaps 80 kHz or so.

The IPG 10 can also communicate data back to the external charger 40 or 60, and this can occur in different manners. As explained in the above-referenced 2017/0361113 publication, the IPG 10 may employ reflected impedance modulation to transmit data to the charger, which is sometimes known in the art as Load Shift Keying (LSK), and which involves modulating the impedance of the charging coil 30 with data bits provided by the IPG 10's control circuitry 86. The IPG may also use a communications channel separate from that used to provide power to transmit data to the charger, although such alternative channel and the antenna required are not shown for simplicity. The charger 40 or 60 can include demodulation circuitry 68 to recover the transmitted data, and to send such data to the charger's control circuitry 72. Such data as telemetered from to the charger 40/60 from the IPG 10 can include information useful for the charger to know during charging, such as the IPG's temperature (as sensed by temperature sensor 87, e.g., thermistor), the voltage Vbat of the IPG's battery 14, or the charging current Ibat provided to the battery. Charger 40/60 can use such telemetered data to control production of the magnetic field 55, such as by increasing or decreasing the magnitude of the magnetic field 55 (by increasing or decreasing Icharge), or by starting or stopping generation of the magnetic field 55 altogether. As explained in the above-referenced 2017/0361113 publication, the charger 40/60 may also be used to determine the alignment of the charging coil 44/66 to the IPG 10, and may include alignment indicators (LEDs or sounds) that a user can review to determine how to reposition the charger to be in better alignment with the IPG 10 for more efficient power transfer.

In addition to communicating data to the charger, the IPG is typically also configured to communicate with one or more other external devices. An example of such other external devices is a patient's external remote controller (RC). The RC can be as described in U.S. Patent Application Publication 2015/0080982 or U.S. Patent No. 11,000,688, for example, and may comprise a stand-alone dedicated controller configured to work with the IPG 10. The RC may also comprise a general purpose mobile electronics device such as a smart phone, which has been programmed with a Medical Device Application (MDA) allowing it to work as a wireless controller for the IPG 10, as described in U.S. Patent Application Publication 2015/0231402. The RC typically includes a user interface, including means for entering commands (e.g., buttons or icons) and a display. The RC's user interface enables a patient to adjust stimulation parameters.

The IPG 10 can include an antenna allowing it to communicate bi-directionally with external devices, such as the RC. The antenna can be the same coil used for charging or an additional coil, and communication can be by LSK, as described above, for example. The IPG 10 may also include a Radio-Frequency (RF) antenna. The antenna may be within the header 23 or within the case 12. The RF antenna may comprise a patch, slot, or wire, and may operate as a monopole or dipole. The RF antenna may communicate using far-field electromagnetic waves, and may operate in accordance with any number of known RF communication standards, such as Bluetooth, Bluetooth Low Energy (BTE), Zigbee, MICS, and the like.

A patient implanted with an IPG typically spends a significant amount of time charging the IPG's rechargeable battery. Inefficient charging, such as charging their device too often or not often enough, or charging with the charger improperly aligned with the IPG, can cause several problems. One problem is that the patient may simply spend more time than necessary charging. Another problem is that improper charging may adversely effect battery life. Still another problem is that if the patient waits too long to charge their battery, their battery might run out of charge. Accordingly, there is a need in the art for detecting when a patient is not practicing efficient charging so that the patient can be encouraged and instructed. US2010222847 discloses a system and a method for monitoring recharging of a rechargeable battery of an implantable pulse generator.

### SUMMARY

The invention is defined by the independent claims. Aspects of this disclosure relate to a cloud-based system for monitoring recharging of a rechargeable battery of an implantable pulse generator (IPG), the system comprising: a remote server configured to: receive via internet, data indicative of one or more measurements obtained from the IPG during the recharging, use the data to determine if one or more recharging efficiency metrics is outside of a predetermined range, and if one or more recharging efficiency metrics is outside of the predetermined range, send an alert via internet to the patient and/or a clinician. According to some embodiments, receiving data indicative of one or more measurements obtained from the IPG during the recharging, comprises receiving the data from a patient's remote controller (RC) associated with the IPG. According to some embodiments, the RC comprises a smartphone. According to some embodiments, the smartphone comprises a medical device application (MDA) configured to periodically send the data to the remote server. According to some embodiments, the data indicative of one or more measurements obtained from the IPG during the recharging comprises one or more of: time stamps, IPG battery voltage measurements, IPG temperatures, and IPG battery charge currents. According to some embodiments, the one or more recharging efficiency metrics comprises a duration of charging. According to some embodiments, the one or more recharging efficiency metrics comprises a frequency of charging. According to some embodiments, the one or more recharging efficiency metrics comprises battery health. According to some embodiments, the one or more recharging efficiency metrics comprises alignment of an external charging coil with a charging coil configured within the IPG. According to some embodiments, determining alignment of the external charging coil with the charging coil comprises using temperature data from the IPG. According to some embodiments, the one or more recharging efficiency metrics comprises an initial battery voltage value when charging is initiated. According to some embodiments, determining if one or more recharging efficiency metrics is outside of a predetermined range comprises identifying a charging session using the data. According to some embodiments, identifying a charging session is based on one or more of identifying a change in battery voltage as a function of time, identifying a charging current, and identifying the presence of an external magnetic field. According to some embodiments, sending an alert via internet to the patient and/or a clinician comprises sending an alert to the patient's RC.

Also disclosed herein is a method for monitoring recharging of a rechargeable battery of an implantable pulse generator (IPG), the method comprising: at a remote server, receiving via internet, data indicative of one or more measurements obtained from the IPG during the recharging, using the data to determine if one or more recharging efficiency metrics is outside of a predetermined range, and if one or more recharging efficiency metrics is outside of the predetermined range, sending an alert via internet to the patient and/or a clinician. According to some embodiments, receiving data indicative of one or more measurements obtained from the IPG during the recharging, comprises receiving the data from a patient's remote controller (RC) associated with the IPG. According to some embodiments, the RC comprises a smartphone. According to some embodiments, the smartphone comprises a medical device application (MDA) configured to periodically send the data to the remote server. According to some embodiments, the data indicative of one or more measurements obtained from the IPG during the recharging comprises one or more of: time stamps, IPG battery voltage measurements, IPG temperatures, and IPG battery charge currents. According to some embodiments, the one or more recharging efficiency metrics comprises a duration of charging. According to some embodiments, the one or more recharging efficiency metrics comprises a frequency of charging. According to some embodiments, the one or more recharging efficiency metrics comprises battery health. According to some embodiments, the one or more recharging efficiency metrics comprises alignment of an external charging coil with a charging coil configured within the IPG. According to some embodiments, determining alignment of the external charging coil with the charging coil comprises using temperature data from the IPG. According to some embodiments, the one or more recharging efficiency metrics comprises an initial battery voltage value when charging is initiated. According to some embodiments, determining if one or more recharging efficiency metrics is outside of a predetermined range comprises identifying a charging session using the data. According to some embodiments, identifying a charging session is based on one or more of identifying a change in battery voltage as a function of time, identifying a charging current, and identifying the presence of an external magnetic field. According to some embodiments, sending an alert via internet to the patient and/or a clinician comprises sending an alert to the patient's RC.

The invention may also reside in the form of a programmed external device (via its control circuitry) for carrying out the above methods, a programmed IPG (via its control circuitry) for carrying out the above method, a system including a programmed external device and IPG for carrying out the above methods, or as a computer readable media for carrying out the above methods stored in an external device, IPG, remote server, or the like.

### BRIEF DESCRIPTION OF THE DRAWINGS

Figures 1A and 1B show different views of an implantable pulse generator, a type of implantable pulse generator (IPG), in accordance with the prior art.
Figures 2A and 2B show different examples of external charger used to wirelessly charge a battery in an IPG or to provide power to the IPG.
Figure 3 shows relevant charging circuitry in the external chargers and the IPG, in accordance with the prior art.
Figure 4 shows a distributed system for monitoring how a patient charges their IPG.
Figure 5 shows an example of a charging data log.
Figure 6 shows an embodiment of an algorithm for evaluating charging frequency.
Figure 7 shows an embodiment of algorithms for monitoring charging behaviors.

### DETAILED DESCRIPTION

The present disclosure is directed to methods and systems for detecting sub-optimal or inefficient charging practices. Examples of sub-optimal or inefficient charging practices include charging too often, not often enough, and/or charging with improper alignment of the charger with respect to the IPG's receiving coil. The methods and systems may alert the patient when poor charging practices are detected and may provide informational materials, for example, to encourage the patient to improve. Sub-optimal charging may also indicate problems with battery health or with the stimulation program(s) being implemented, and the instant methods and systems may bring such problems to the patient's and/or clinician's attention.

Figure 4 illustrates an embodiment of a system 400 comprising the patient's IPG 10, which may communicate with their RC 402. As mentioned above, the RC 402 may comprise a dedicated, stand-alone RC or may comprise a personal computing/communications device, such as a smartphone programmed with a Medical Device Application (MDA) 404. According to some embodiments, the IPG may transmit information to a stand-alone RC, which then transmits the information to a general personal computing device, such as a smartphone. The RC 402 (stand-alone or generalized) may communicate with remote locations via the internet or other network 406, for example, using WiFi, cellular, or other communications methods. For example, the RC may communicate with a one or more remote servers 408, which may be located at a remote data center. The RC may also communicate with a clinician's office 410. Likewise, the server 408 and the clinician's office 410 may communicate with each other via the network 406.

In the illustrated embodiment, the IPG 10 is configured to record certain parameters/data and measurements related to charging in a charging log 412, which will be explained in more detail below. According to some embodiments, the information contained within the charging log may be transmitted to the RC 402 and then to the remote server 408 via the internet. According to some embodiments, the IPG may automatically transmit the charging log to the patient's RC upon completion of each charging session. According to other embodiments, the RC may prompt the patient to cause the IPG data to be uploaded to the RC. In either case, the RC may transmit the IPG data/charging log to the remote server 408. One or more charging algorithms 414 at the remote server 408 may use the information from the charging log to determine information about the patient's charging practices, as described in more detail below. Stated differently, the algorithm(s) 414 use the data from the IPG to determine values for one or more charging efficiency metrics. Examples of charging efficiency metrics are described in more detail below, but may include how often the patient recharges their IPG, how low the battery is allowed to get between recharging sessions, battery health, alignment of the charger with the IPG, etc.

According to some embodiments, the algorithm(s) 414 may be embodied as instructions stored on non-transitory computer-readable media at the server. Such non-transitory media may include one or more non-transitory computer-readable storage mediums including, non-volatile memory, magnetic disks (fixed, floppy, and removable) and tape, optical media such as CD-ROMs and digital video disks (DVDs), and semiconductor memory devices such as Electrically Programmable Read-Only Memory (EPROM), Electrically Erasable Programmable Read-Only Memory (EEPROM), and USB or thumb drive. The instructions, when executed, perform the algorithms described herein. One skilled in the art will additionally recognize that execution of the instructions can be facilitated by control circuitry such as a microprocessor, microcomputer, an FPGA, other digital logic structures, etc., which is capable of executing programs in a computing device.

In response to the algorithm's determinations, information 416, such as alerts, instructional information, and the like, may be transmitted back to the patient's RC, as explained in more detail below. The server 408 may send alerts/information to the clinician's office 410, which may prompt the clinician to take certain actions, such as transmitting information 416 to the patient's RC, contacting the patient, and/or remotely reprogramming the patient's stimulation parameters, as described in more detail below. It should be noted that the system 400 may be configured differently than illustrated in Figure 4. For example, the algorithm 414 may be performed in whole or in part by the IPG 10 and/or the RC 402, in which case non-transitory computer-readable media and control circuitry of the respective devices will be used.

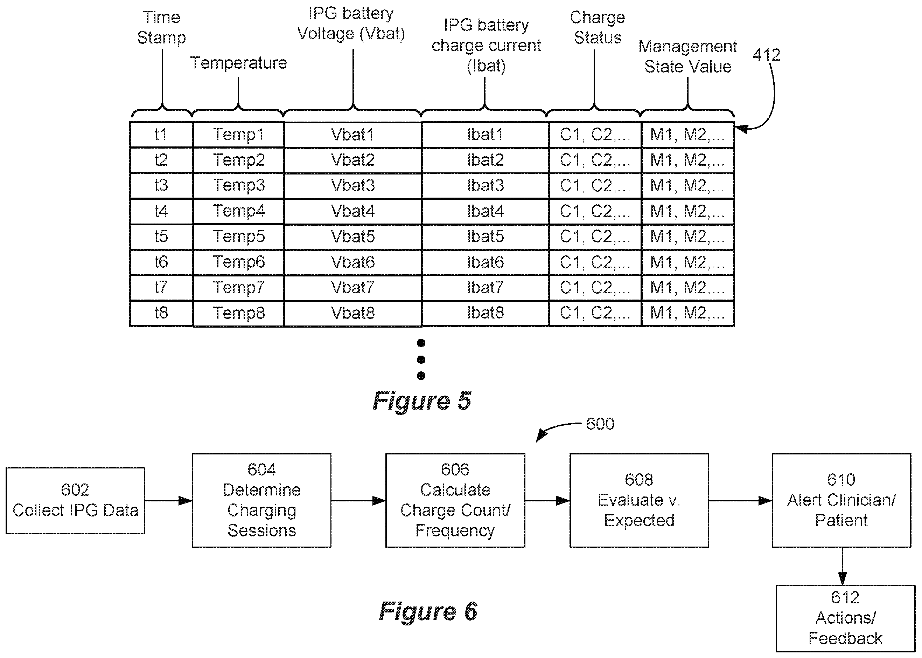

Figure 5 illustrates an embodiment of a charging log 412. As mentioned above, the charging log 412 may be recorded in a memory of the IPG. Alternatively, the IPG may telemeter the relevant data to the patient's RC and the charging log may be recorded in the RC 402. Still alternatively, the relevant data may be transmitted to a remote location, such as the remote server 408 and the charging log may be recorded there.

According to some embodiments, relevant data can be recorded into the charging log 412 periodically, when charging is detected. For example, when charging is detected, data may be recorded every ten minutes, every five minutes, or every one minute. Data may be recorded for some period of time once charging is completed. For example, assume that once charging is detected, data is collected every five minutes. Once charging has stopped, and additional six data points may be collected, for example, meaning that data collection continues for an additional thirty minutes after charging has stopped.

Charging may be detected by sensing the presence of a charging magnetic field. For example, the IPG may be configured with a sensor, such as a Reed sensor, a Hall effect sensor, or the like. Charging may also be detected by determining that the charge on the battery is increasing, and/or by detecting a non-zero charging current Ibat (Fig. 3). In any case, detection of charging may cue the IPG to begin collecting relevant data for the charging log. The completion of charging may be determined by determining that the charging field is no longer present, that the charging current Ibat is no longer present, or that the battery voltage Vbat is no longer increasing, for example.

As illustrated in Figure 5, the charging log 412 may include various data related to the charging of the IPG's battery. The illustrated charging log tabulates time stamps, and for each time stamp a temperature, a battery voltage Vbat, and a battery charge current Ibat. Embodiments of the charging log may also include one or more additional values, referred to herein as charge status values. Examples of charge status values (C1, C2, ...) include indications that a charger is present, that a hardware overvoltage protection status has been reached, firmware overvoltage protection status has been reached, and/or that an overtemperature status has been reached, etc. Embodiments of the charging log may also include one or more additional values, referred to herein as management state values (M1, M2, ... ). Examples of management state values may include indications of the presence (or absence) of a charging field, constant current being reached, constant voltage being reached, end of charge, end of charge lock-out, etc.

Figure 6 illustrates an algorithm 600 (which may be one example of the charging algorithm 414 (Fig. 4)) for evaluating the frequency (or number of times) a patient charges their IPG. As described above, the algorithm may be performed at the server 408 (Fig. 4) based on data in the charging log 412 received from the IPG. Alternatively, the algorithm 600 may be performed by the control circuitry of the IPG or the control circuitry of the patient's RC (including being performed by a MDA on the patient's smartphone, for example).

At step 602, the algorithm collects data from the IPG. For example, the algorithm may receive the charging log 412 from IPG. At step 604, the algorithm may parse the received log/data to identify charging sessions. For example, the algorithm may look for beginning of charging session data and end of charging session data, as described above. At step 606, the algorithm can calculate the number of charging sessions, the number of charging sessions within a given period of time (i.e., a charging frequency), and/or the duration of charging sessions. For example, the algorithm may determine the number of times per week the patient recharges their IPG. Alternatively, the algorithm may determine the length of time between charging sessions. The algorithm can also determine the durations of the charging sessions. At step 608, the algorithm may compare the number of charges/charging frequency to an expected value to determine if the patient is charging their device often enough (or too often). For example, the algorithm may compare the amount of charging to an expected threshold value or a range between upper and lower threshold values. The expected charging times may be predicted, for example, based on knowledge of the particular IPG, the particular battery, etc., as well as a calculation of the energy required for the particular stimulation program(s) the patient is using. U.S. Patent No. 9,327,135 describes methods of predicting charging frequency/duration requirements based on information about the IPG's stimulation parameter/programs. The expected charging times may also be based on accumulated historic data for the patient's behavior, as well as accumulated data from many patients.

Based on the comparison of the recorded charging behavior with expected values/ranges, the algorithm may alert the clinician and/or the patient (Step 610). The algorithm and/or the clinician may use the system to provide feedback to the patient or to take other actions (Step 612). If the charging behavior comports with expected/recommended practice, the algorithm may issue the patient a message informing them as such, and encouraging them to continue, for example. The clinician may be updated as to the behavior for their records. However, if the charging behavior deviates from recommended practice, then the algorithm may cause the server to send the patient a message letting them know of the problem. The message may be a reminder to charge more or less often, as the case may be. The message may include training materials, videos, links to web-based information, and the like. Likewise, the clinician may be notified so they can provide corrective instruction. Also, if the clinician notices that the patient's battery is running low more often than expected, the clinician may reprogram the patient's IPG to use a less power-intensive stimulation program. The clinician may instruct the patient to make an appointment so that the IPG can be reprogrammed. Alternatively, according to some embodiments, the clinician may use the system 400 (Fig. 4) to remotely reprogram the IPG. Still alternatively, the patient may be instructed how to reprogram their IPG using their RC. It should be noted here that communications from the algorithm to the patient and/or from the clinician to the patient may be delivered through the patient's RC, smartphone apps (such as the MDA), via text message, through a patient portal, etc.

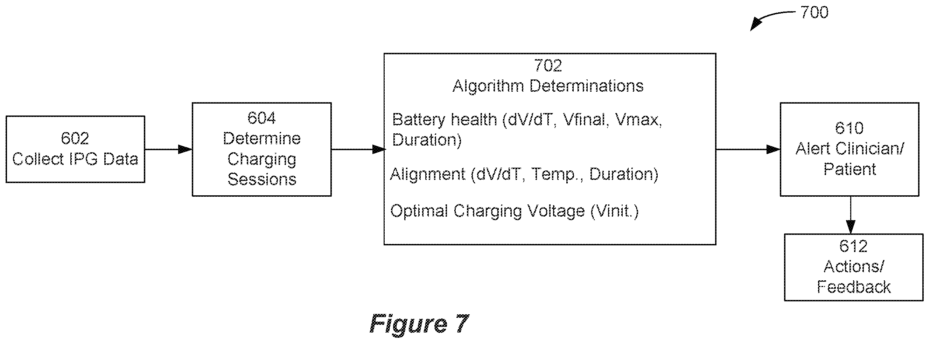

Algorithm 600 (Fig. 6) is configured to evaluate how often a patient charges their IPG. Figure 7 illustrates a more generalized algorithm 700 (which, again, is an embodiment of a charging algorithm 416 (Fig. 4)) that can use the data contained the charging log 412 to determine one or more aspects of charging. Steps 602 and 604 of obtaining data from the IPG and determining charging sessions may be the same as described above, with respect to the algorithm 600 (Fig. 6). Likewise, the steps of alerting the clinician and/or patient 610 and of providing feedback 612 are generally the same as described above. The content of the alerts, information, and/or feedback will depend on the specifics of the determinations made by the algorithm 700, as described further below.

At step 702, the algorithm may make one or more determinations concerning the charging of the IPG, such as the ones enumerated in the figure. According to some embodiments, step 702 may involve determining/predicting the health of the IPG's battery. According to some embodiments, the battery's health may be evaluated based on the voltage v. time curve (dV/dT) during charging. According to some embodiments, the battery's health may be evaluated based on the final battery voltage at the end of a charging session (Vfinal) or the maximum voltage (Vmax) of the battery. According to some embodiments, the battery's health may be evaluated based on the duration of charging required to reach Vmax. Any of these data may be evaluated by comparing the values to absolute expected values or thresholds. For example, if any or all of dV/dT, Vmax, or Vfinal are less than an expected or threshold value, then a decline of the battery's health may be suspected. Likewise, a decline of the battery's health may be indicated if the duration of the charging time required for the battery to fully charge exceeds an expected value or a threshold. According to some embodiments, changes in values compared to historically recorded values for these data may indicate a decline in battery health. For example, if dV/dT, Vmax, and/or Vfinal decrease over time, or if the charging duration increases over time, that may indicate failing battery health. If the algorithm determines/predicts that the health of the battery is declining, then the algorithm may alert the patient and/or the clinician as such.

According to some embodiments, at step 702 the algorithm may determine if the patient is properly aligning their charger with their IPG during charging. If the patient inconsistently aligns their charger with the IPG, the voltage v. time curve (dV/dT) may vary during a charging session and/or may vary from charging session to charging session. Likewise, higher than expected temperature during charging (i.e., thermistor value) may indicate poor alignment. Longer than expected charging durations may also indicate poor alignment. Any of dV/dT, temperature, and/or charging duration may be compared expected or threshold values to diagnose poor alignment. If poor alignment is detected, the algorithm may alert the patient. According to some embodiments, the algorithm may alert the patient during the course of a charging session if one or more of these values changes. For example, if the data indicates that charging is efficient at the beginning of a charging session but then changes, the algorithm may send an alert to the patient asking them if they moved the position of their charger or otherwise changed positions. According to some embodiments, the algorithm may provide training information to the patient, such as links to videos or other instructions regarding proper alignment. According to some embodiments, the algorithm may notify the clinician of improper charging alignment so that they can address the issue during the patient's next visit.

According to some embodiments, the algorithm can determine if the patient is routinely charging the IPG's battery at the proper time, that is, when the battery voltage is at the proper stage of depletion. As known to those of skill in the art, repeatedly charging a rechargeable battery when the battery is already substantially charged may speed the degradation of the battery. Also, repeatedly allowing the battery to completely drain may be damaging. According to some embodiments, the algorithm may track the voltages at a beginning of charging sessions to determine the typical state of the battery when the patient begins recharging. In other words, the algorithm may determine if the patient tends to charge the battery too soon, i.e., when there is substantial charge remaining, or too late, i.e., when the battery is almost dead. For example, the algorithm may provide alerts to the patient if they are recharging the battery when there is greater than 80 % or less than 20 % charge remaining.

According to some embodiments the charging algorithm 416 may make determinations based on the data received from the IPG (e.g., from the charging log 412) combined with data/information from other sources. For example, the algorithm may be configured to issue charging alerts and/or suggest optimum times for charging sessions, based on information concerning the patient's activities, schedule, routine, lifestyle, etc., which the patient may provide using the MDA of their RC, for example. Alternatively, patient activity information may be determined based on sensor data, such as accelerometer data. The accelerometer may be configured as part of the IPG or may be another device, such as a wearable accelerometer. The algorithm may use such information to suggest charging times that are convenient for the patient, such as when the patient is awake and not asleep and also when the patient is not in the middle of an activity. According to some embodiments, the algorithm may use historic data collected from the patient to derive such suggestions.

Although particular embodiments of the present invention have been shown and described, it should be understood that the above discussion is not intended to limit the present invention to these embodiments. It will be obvious to those skilled in the art that various changes and modifications may be made without departing from the scope of the present invention.

## Claims

1. A cloud-based system (400) for monitoring recharging of a rechargeable battery (14) of an implantable pulse generator, IPG, (10) configured to be implanted in a patient, the system (400) comprising:
a remote server (408) configured to:
receive, via internet, data indicative of one or more measurements obtained from the IPG (10) during the recharging,
use the data to determine if one or more recharging efficiency metrics is outside of a predetermined range, and
if one or more recharging efficiency metrics is outside of the predetermined range, send an alert, via internet, to the patient and/or a clinician, wherein the one or more recharging efficiency metrics comprises alignment of an external charging coil (66) with a charging coil (30) configured within the IPG (10), and wherein determining alignment of the external charging coil (66) with the charging coil (30) comprises using temperature data from the IPG (10).

2. The system of claim 1, wherein receiving data indicative of one or more measurements obtained from the IPG (10) during the recharging, comprises receiving the data from a patient's remote controller, RC, (402) associated with the IPG (10).

3. The system of claim 2, wherein the RC (402) comprises a smartphone.

4. The system of claim 3, wherein the smartphone comprises a medical device application, MDA, configured to periodically send the data to the remote server.

5. The system of any of claims 1-4, wherein the data indicative of one or more measurements obtained from the IPG (10) during the recharging comprises one or more of: time stamps, IPG battery voltage measurements, IPG temperatures, and IPG battery charge currents.

6. The system of any of claims 1-5, wherein the one or more recharging efficiency metrics comprises a duration of charging.

7. The system of any of claims 1-6, wherein the one or more recharging efficiency metrics comprises a frequency of charging.

8. The system of any of claims 1-7, wherein the one or more recharging efficiency metrics comprises battery health.

9. The system of any of claims 1-8, wherein the one or more recharging efficiency metrics comprises an initial battery voltage value when charging is initiated.

10. The system of any of claims 1-9, wherein determine if one or more recharging efficiency metrics is outside of a predetermined range comprises identifying a charging session using the data.

11. The system of claim 10, wherein identifying a charging session is based on one or more of identifying a change in battery voltage as a function of time, identifying a charging current, and identifying the presence of an external magnetic field.

12. The system of any of claims 1-11, wherein sending an alert via internet to the patient and/or a clinician comprises sending an alert to the patient's RC (402).

13. A method for monitoring recharging of a rechargeable battery (14) of an implantable pulse generator, IPG, (10) configured to be implanted in a patient, the method comprising:
at a remote server (408), receiving, via internet, data indicative of one or more measurements obtained from the IPG (10) during the recharging,
using the data to determine if one or more recharging efficiency metrics is outside of a predetermined range, and
if one or more recharging efficiency metrics is outside of the predetermined range, sending an alert, via internet, to the patient and/or a clinician, wherein the one or more recharging efficiency metrics comprises alignment of an external charging coil (66) with a charging coil (30) configured within the IPG (10), and wherein determining alignment of the external charging coil (66) with the charging coil (30) comprises using temperature data from the IPG (10).

## Patentansprüche

1. Cloudbasiertes System (400) zum Überwachen des Ladevorgangs einer wiederaufladbaren Batterie (14) eines implantierbaren Impulsgenerators, IPG, (10), der dafür konfiguriert ist, in einem Patienten implantiert zu werden, wobei das System (400) aufweist:
einen Remote-Server (408), der dafür konfiguriert ist:
über das Internet Daten zu empfangen, die eine oder mehrere Messwerte anzeigen, die während des Ladevorgangs vom IPG (10) erhalten werden;
die Daten zu verwenden, um festzustellen, ob eine oder mehrere Ladeeffizienzkennzahlen außerhalb eines vorgegebenen Bereichs liegen; und,
falls eine oder mehrere Ladeeffizienzkennzahlen außerhalb des vorgegebenen Bereichs liegen, eine Warnmeldung über das Internet an den Patienten und/oder einen Kliniker zu übertragen, wobei die eine oder die mehreren Ladeeffizienzkennzahlen die Ausrichtung einer externen Ladespule (66) mit einer im IPG (10) konfigurierten Ladespule (30) aufweisen, und wobei das Bestimmen der Ausrichtung der externen Ladespule (66) mit der Ladespule (30) das Verwenden von Temperaturdaten vom IPG (10) aufweist.

2. System nach Anspruch 1, wobei das Empfangen von Daten, die eine oder mehrere während des Aufladens vom IPG (10) erhaltene Messwerte anzeigen, das Empfangen der Daten von einer dem IPG (10) zugeordneten Fernbedienung, RC, (402) eines Patienten aufweist.

3. System nach Anspruch 2, wobei die RC (402) ein Smartphone aufweist.

4. System nach Anspruch 3, wobei das Smartphone eine medizinische Geräteanwendung, MDA, aufweist, die dafür konfiguriert ist, die Daten periodisch an den Remote-Server zu übertragen.

5. System nach einem der Ansprüche 1 bis 4, wobei die Daten, die eine oder mehrere Messwerte anzeigen, die während des Ladevorgangs vom IPG (10) erhalten werden, eines oder mehrere der folgenden Elemente aufweisen: Zeitstempel, IPG-Batteriespannungsmesswerte, IPG-Temperaturen und IPG-Batterieladeströme.

6. System nach einem der Ansprüche 1 bis 5, wobei die eine oder die mehreren Ladeeffizienzkennzahlen eine Ladedauer aufweisen.

7. System nach einem der Ansprüche 1 bis 6, wobei die eine oder die mehreren Ladeeffizienzkennzahlen eine Ladefrequenz aufweisen.

8. System nach einem der Ansprüche 1 bis 7, wobei die eine oder die mehreren Ladeeffizienzkennzahlen einen Batteriebetriebszustand aufweisen.

9. System nach einem der Ansprüche 1 bis 8, wobei die eine oder die mehreren Ladeeffizienzkennzahlen einen anfänglichen Batteriespannungswert bei Beginn des Ladevorgangs aufweisen.

10. System nach einem der Ansprüche 1 bis 9, wobei das Bestimmen, ob eine oder mehrere Ladeeffizienzkennzahlen außerhalb eines vorgegebenen Bereichs liegen, das Identifizieren einer Ladesitzung unter Verwendung der Daten aufweist.

11. System nach Anspruch 10, wobei das Identifizieren einer Ladesitzung auf einem oder mehreren der folgenden Schritte basiert: Identifizieren einer Änderung der Batteriespannung als Funktion der Zeit, Identifizieren eines Ladestroms und Identifizieren des Vorhandenseins eines externen Magnetfelds.

12. System nach einem der Ansprüche 1 bis 11, wobei das Übertragen einer Warnmeldung über das Internet an den Patienten und/oder einen Kliniker das Übertragen einer Warnmeldung an die RC (402) des Patienten aufweist.

13. Verfahren zum Überwachen des Ladevorgangs einer wiederaufladbaren Batterie (14) eines implantierbaren Impulsgenerators, IPG, (10), der dafür konfiguriert ist, in einem Patienten implantiert zu werden, wobei das Verfahren aufweist:
Empfangen von Daten, die einen oder mehrere Messwerte anzeigen, die während des Ladevorgangs vom IPG (10) erhalten werden, über das Internet an einem Remote-Server (408);
Verwenden der Daten zum Feststellen, ob eine oder mehrere Ladeeffizienzkennzahlen außerhalb eines vorgegebenen Bereichs liegen; und,
falls eine oder mehrere Ladeeffizienzkennzahlen außerhalb des vorgegebenen Bereichs liegen, Übertragen einer Warnmeldung über das Internet an den Patienten und/oder einen Kliniker, wobei die eine oder die mehreren Ladeeffizienzkennzahlen die Ausrichtung einer externen Ladespule (66) mit einer innerhalb des IPG (10) angeordneten Ladespule (30) aufweisen, und wobei das Bestimmen der Ausrichtung der externen Ladespule (66) mit der Ladespule (30) das Verwenden von Temperaturdaten vom IPG (10) aufweist.

## Revendications

1. Système basé sur le nuage (400) pour surveiller la recharge d'une batterie rechargeable (14) d'un générateur d'impulsions implantable, IPG, (10) configuré pour être implanté chez un patient, le système (400) comprenant :
un serveur distant (408) configuré pour :
recevoir via Internet des données indicatives d'une ou plusieurs mesures obtenues à partir de l'IPG (10) pendant la recharge,
utiliser les données pour déterminer si une ou plusieurs métriques d'efficacité de recharge se trouvent en dehors d'une plage prédéterminée, et
si une ou plusieurs métriques d'efficacité de recharge se trouvent en dehors de la plage prédéterminée, envoyer une alerte via Internet au patient et/ou à un clinicien, dans lequel les une ou plusieurs métriques d'efficacité de recharge comprennent l'alignement d'une bobine de charge externe (66) avec une bobine de charge (30) configurée à l'intérieur de l'IPG (10), et dans lequel la détermination de l'alignement de la bobine de charge externe (66) avec la bobine de charge (30) comprend l'utilisation de données de température provenant de l'IPG (10).

2. Système selon la revendication 1, dans lequel la réception de données indicatives d'une ou plusieurs mesures obtenues à partir de l'IPG (10) pendant la recharge comprend la réception des données provenant d'une télécommande, RC, (402) d'un patient associée à l'IPG (10).

3. Système selon la revendication 2, dans lequel la RC (402) comprend un téléphone intelligent.

4. Système selon la revendication 3, dans lequel le téléphone intelligent comprend une application de dispositif médical, MDA, configurée pour envoyer périodiquement les données au serveur distant.

5. Système selon l'une des revendications 1 à 4, dans lequel les données indicatives d'une ou plusieurs mesures obtenues à partir de l'IPG (10) pendant la recharge comprennent un ou plusieurs parmi : des horodatages, des mesures de tension de batterie d'IPG, des températures d'IPG et des courants de charge de batterie d'IPG.

6. Système selon l'une des revendications 1 à 5, dans lequel les une ou plusieurs métriques d'efficacité de recharge comprennent une durée de charge.

7. Système selon l'une des revendications 1 à 6, dans lequel les une ou plusieurs métriques d'efficacité de recharge comprennent une fréquence de charge.

8. Système selon l'une des revendications 1 à 7, dans lequel les une ou plusieurs métriques d'efficacité de recharge comprennent un état de santé de batterie.

9. Système selon l'une des revendications 1 à 8, dans lequel les une ou plusieurs métriques d'efficacité de recharge comprennent une valeur de tension de batterie initiale lorsque la charge est initiée.

10. Système selon l'une des revendications 1 à 9, dans lequel le fait de déterminer si une ou plusieurs métriques d'efficacité de recharge se trouvent en dehors d'une plage prédéterminée comprend l'identification d'une session de charge à l'aide des données.

11. Système selon la revendication 10, dans lequel l'identification d'une session de charge est basée sur une ou plusieurs parmi l'identification d'un changement de tension de batterie en fonction du temps, l'identification d'un courant de charge et l'identification de la présence d'un champ magnétique externe.

12. Système selon l'une des revendications 1 à 11, dans lequel l'envoi d'une alerte via Internet au patient et/ou à un clinicien comprend l'envoi d'une alerte à la RC du patient (402).

13. Procédé pour surveiller la recharge d'une batterie rechargeable (14) d'un générateur d'impulsions implantable, IPG, (10) configuré pour être implanté chez un patient, le procédé comprenant :
sur un serveur distant (408), la réception via Internet de données indicatives d'une ou plusieurs mesures obtenues à partir de l'IPG (10) pendant la recharge,
l'utilisation des données pour déterminer si une ou plusieurs métriques d'efficacité de recharge se trouvent en dehors d'une plage prédéterminée, et
si une ou plusieurs métriques d'efficacité de recharge se trouvent en dehors de la plage prédéterminée, l'envoi d'une alerte via Internet au patient et/ou à un clinicien, dans lequel les une ou plusieurs métriques d'efficacité de recharge comprennent l'alignement d'une bobine de charge externe (66) avec une bobine de charge (30) configurée à l'intérieur de l'IPG (10), et dans lequel la détermination de l'alignement de la bobine de charge externe (66) avec la bobine de charge (30) comprend l'utilisation de données de température provenant de l'IPG (10).
